(19)

Europäisches
Patentamt
European
Patent Office
Office européen
des brevets

(11) **EP 3 053 516 A1**

(12) **EUROPEAN PATENT APPLICATION**

(43) Date of publication:
**10.08.2016 Bulletin 2016/32**

(21) Application number: **16154055.4**

(22) Date of filing: **03.02.2016**

(51) Int Cl.:
*A61B 5/00* (2006.01)          *A61B 5/01* (2006.01)
*G01K 17/02* (2006.01)          *G01K 17/12* (2006.01)
*G01K 17/20* (2006.01)          *G01K 13/00* (2006.01)
*G01J 5/02* (2006.01)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**MA MD**

(30) Priority: **05.02.2015 JP 2015020899**

(71) Applicant: **Seiko Epson Corporation**
**Tokyo 163 (JP)**

(72) Inventors:
• **Ikeda, Akira**
**Nagano, 392-8502 (JP)**
• **Hokari, Ryuji**
**Nagano, 392-8502 (JP)**

(74) Representative: **Hoffmann Eitle**
**Patent- und Rechtsanwälte PartmbB**
**Arabellastraße 30**
**81925 München (DE)**

(54) **VAPORIZATION HEAT QUANTITY MEASURING DEVICE, BIOLOGICAL INFORMATION MEASURING DEVICE, AND ELECTRONIC APPARATUS**

(57)    In an electronic apparatus, a contact thermometer contacts a skin surface of a user and measures a first temperature of the skin surface. A radiation thermometer measures a second temperature of the skin surface of the user. A vaporization heat quantity calculating unit calculates a quantity of heat of vaporization, using the first temperature and the second temperature. The calculation of the quantity of heat of vaporization is performed by executing predetermined vaporization heat quantity computing processing in which the first temperature and the second temperature are variables.

FIG.12

**Description**

BACKGROUND

1. Technical Field

The present invention relates to a vaporization heat quantity measuring device and the like which measures the quantity of heat of vaporization.

2. Related Art

The body temperature of a human body is maintained by the heat dissipated from the human body. The metabolism to maintain the body temperature is called basal metabolism. That is, the metabolic rate can be learned by measuring dissipation from the human body. Heat dissipation is generated by the perspiration function, which is a physiological function of the human body. Specifically, the human body perspires through sweat glands in the skin and vaporizes (evaporates) water from the skin surface, thus dissipating heat outside the body as heat of vaporization (evaporative heat loss).

JP-A-2011-120917 discloses a technique of finding physiological information or context information from the wearer's body. For example, the publication discloses a method of acquiring heart rate, pulse rate, respiratory rate, heat flow, oxygen consumption and the like and thus finding the calories burned and basal metabolic rate.

While JP-A-2011-120917 discloses a configuration for detecting the amount of perspiration by measuring the electrical skin resistance, it does not disclose a technique of finding the evaporative heat loss, that is, the quantity of heat of vaporization due to perspiration. Since how much of the perspiration water is evaporated varies depending on the humidity and water vapor pressure in the external environment, there are cases where the quantity of heat of vaporization cannot be accurately measured on the basis of the amount of perspiration alone, for example, at the time of exercise or in hot environment or the like where the person perspires a lot.

This problem is not limited to the case where the measuring target is a human body. The same applies to measuring the quantity of heat of vaporization due to perspiration of other organisms and also to measuring the quantity of heat of vaporization in objects which generate heat of vaporization.

SUMMARY

An advantage of some aspects of the invention is that the quantity of heat of vaporization from a measurement target can be measured.

A first aspect of the invention is directed to a vaporization heat quantity measuring device including: a contact thermometer which contacts a surface of a measuring target and measures a temperature of the surface (first temperature) ; a radiation thermometer which measures a second temperature of the surface of the measuring object; and a computing unit which calculates a quantity of heat of vaporization, using the first temperature and the second temperature.

As water evaporates from the surface of the measuring object, heat of vaporization is taken away and therefore the temperature of the surface drops. However, since the portion which the contact thermometer contacts is not exposed to the external environment, a temperature drop is less likely to happen. Therefore, the second temperature measured of the surface of the measuring target is lower than the first temperature measured in contact with the surface. According to the first aspect of the invention, the quantity of heat of vaporization from the measuring target can be measured, using the first temperature and the second temperature of the surface of the measuring target.

A second aspect of the invention is directed to the vaporization heat quantity measuring device according to the first aspect of the invention, in which the computing unit executes predetermined vaporization heat quantity computing processing in which the first temperature and the second temperature are variables, and thus may calculate the quantity of heat of vaporization.

According to the second aspect of the invention, the quantity of heat of vaporization can be calculated by the computing processing using the first temperature and the second temperature.

A third aspect of the invention is directed to the vaporization heat quantity measuring device according to the second aspect of the invention, in which the computing unit executes computing processing in which the quantity of heat of vaporization is calculated on the basis of a linear sum of the first temperature and the second temperature, as the vaporization heat quantity computing processing.

According to the third aspect of the invention, the quantity of heat of vaporization can be calculated on the basis of the linear sum of the first temperature and the second temperature.

A fourth aspect of the invention is directed to the vaporization heat quantity measuring device according to the second aspect of the invention, in which the computing unit executes computing processing in which the quantity of heat of vaporization is calculated on the basis of a sum of squares of the first temperature and the second temperature, as the vaporization heat quantity computing processing.

According to the fourth aspect of the invention, the quantity of heat of vaporization can be calculated on the basis of the sum of squares of the first temperature and the second temperature.

A fifth aspect of the invention is directed to a biological information measuring device including a plurality of the vaporization heat quantity measuring devices according to any of the first to fourth aspects of the invention which measure the quantity of heat of vaporization at different sites on a living body as the measuring target, in which the biological information measuring device measures biological information including at least a metabolic rate of the living body, using the quantity of heat of vaporization measured at each of the sites.

According to the fifth aspect of the invention, the quantity of heat of vaporization can be measured at a plurality of sites of the living body and the metabolic rate of the living body can be measured on the basis of the quantity of heat of vaporization at each site.

A sixth aspect of the invention is directed to a biological information measuring device including: the vaporization heat quantity measuring device according to any of the first to fourth aspects of the invention which measures the quantity of heat of vaporization from a living body as the measuring target; and a heat flow meter which measures a heat flow generated on a surface of the living body, in which the biological information measuring device measures biological information including at least a metabolic rate of the living body, using the quantity of heat of vaporization measured by the vaporization heat quantity measuring device and the heat flow measured by the heat flow meter.

According to the sixth aspect of the invention, the metabolic rate of the living body can be measured on the basis of the quantity of heat of vaporization from the living body and the heat flow generated on the surface of the living body.

A seventh aspect of the invention is directed to an electronic apparatus mounted on a skin surface at a predetermined site of a user by having a band thereof wound on the site, including: a contact thermometer which contacts the skin surface and measures a first temperature of the skin surface; a radiation thermometer which measures a second temperature of the skin surface; and a computing unit which calculates a quantity of heat of vaporization, using the first temperature and the second temperature.

According to the seventh aspect of the invention, an electronic apparatus having advantageous effects similar to those of the first aspect can be achieved.

BRIEF DESCRIPTION OF THE DRAWINGS

The invention will be described with reference to the accompanying drawings, wherein like numbers reference like elements.

FIG. 1 is an external view showing an example of the overall configuration of an electronic apparatus according to a first embodiment.

FIG. 2 is a schematic view showing the positional relation between a contact thermometer, a radiation thermometer and a reflection mirror.

FIG. 3 is a block diagram showing an example of the main functional configuration of the electronic apparatus according to the first embodiment.

FIG. 4 is a block diagram schematically showing an example of the internal configuration of a thermometer unit.

FIG. 5 is a flowchart showing the flow of vaporization heat quantity computing processing according to the first embodiment.

FIG. 6 is a block diagram schematically showing an example of the internal configuration of a thermometer unit according to a modification.

FIG. 7 shows the result of an experiment.

FIG. 8 is a block diagram showing an example of the main functional configuration of an electronic apparatus according to a second embodiment.

FIG. 9 is a flowchart showing the flow of metabolic rate computing processing according to the second embodiment.

FIG. 10 is a block diagram showing an example of the main functional configuration of an electronic apparatus according to a third embodiment.

FIG. 11 is a flowchart showing the flow of metabolic rate computing processing according to the third embodiment.

FIG. 12 is a block diagram showing an example of the main functional configuration of an electronic apparatus according to a fourth embodiment.

FIG. 13 is a flowchart showing the flow of metabolic rate computing processing according to the fourth embodiment.

DESCRIPTION OF EXEMPLARY EMBODIMENTS

Hereinafter, a form of embodiment for carrying out the vaporization heat quantity measuring device and the like according to the invention will be described. However, the invention is not limited to the embodiment described below and the form of embodiment to which the invention is applicable is not limited to the embodiment below, either. In the drawings, the same parts are denoted by the same reference numbers.

First Embodiment

First, as a first embodiment, an electronic apparatus which is mounted on a user and measures the quantity of heat of vaporization due to perspiration (hereinafter also referred to as "quantity of heat of perspiration vaporization") . If the quantity of heat of perspiration vaporization is known, heat dissipation to the outside from the body due to perspiration can be grasped. Heat dissipation from a human body is related to human homeostasis such as thermoregulation, for example. Therefore, knowing this is important for health sciences and medicine.

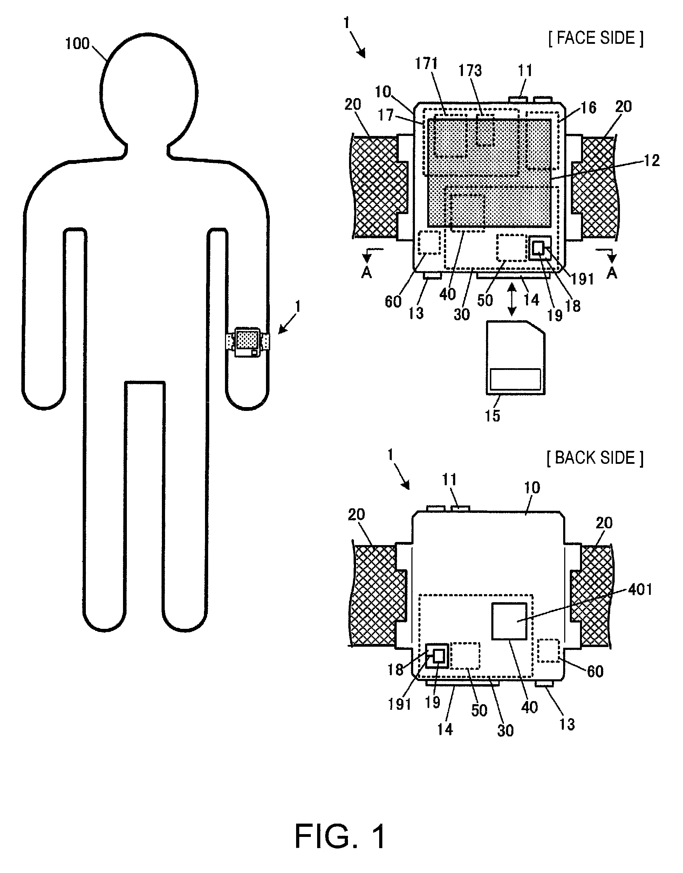

FIG. 1 is an external view showing an example of the overall configuration of an electronic apparatus 1 according to the first embodiment. This electronic apparatus 1 is configured as a wristwatch-type wearable apparatus. When in use, the electronic apparatus 1 is mounted and fixed on the skin surface by causing a band 20 provided on a main body case 10 to be wound around the wrist of a user 100. The form of mounting the apparatus is not limited to the mounting on the skin surface of the wrist and the apparatus may also be mounted on the skin surfaces of other sites such as the neck, upper arm, ankle, chest, and trunk.

The electronic apparatus 1 has an operation switch 11 on a lateral side of the main body case 10, as an operation input unit, and has a touch panel 12 which also functions as an image display unit, on a face side of the main body case 10 (the side facing outward when the apparatus is mounted on the user 100). The user 100 can input various operations such as measurement start operation, using these components.

On a lateral side of the main body case 10, a communication device 13 where a wire cable for communicating with an external device can be attached and removed, and a reader/writer 14 which implements reading data from and writing data into a memory card 15. The main body case 10 also contains a rechargeable built-in battery 16, a control board 17, a thermometer unit 30, and a heat flow meter 60.

The communication device 13 can be materialized by a wireless communication module and an antenna, if it is to have a configuration for performing wireless communication with an external device. The memory card 15 is a removable non-volatile memory in which data is rewritable. As the memory card 15, a rewritable non-volatile memory such as a FeRAM (ferroelectric random access memory) or MRAM (magnetoresistive random access memory) as well as flash memory can be used.

The charging method for the built-in battery 16 can be set suitably according to need. For example, an electrical contact may be separately provided on the back side of the main body case 10, and the main body case 10 may be set in a cradle connected to a household power supply so that the built-in battery 16 can be electrified and charged via the electrical contact and the cradle. Alternatively, contactless wireless charging may be used.

The control board 17 is equipped with a CPU (central processing unit) 171 and a storage medium 173 such as an IC (integrated circuit) memory or hard disk. Also, necessary electronic components such as an ASIC (application specific integrated circuit) and various other integrated circuits can be suitably installed according to need. The electronic apparatus 1 implements various functions such as vaporization heat quantity measurement, by causing the CPU 171 to execute a program stored in the storage medium 173.

The thermometer unit 30 is made up of a contact thermometer 40 and a radiation thermometer 50. The contact thermometer 40 is configured to contact the skin surface and measure its contact temperature and is arranged on the back side of the main body case 10 (the side which contacts the skin surface when the apparatus is mounted on the user 100) in such a way that a sensor surface 401 is exposed. Meanwhile, the radiation thermometer 50 is configured to measure the radiation temperature of the skin surface via the outside air between the skin surface and the radiation thermometer 50 and is arranged in a suitable position inside the main body case 10.

Here, the main body case 10 has a penetration hole 18 vertically penetrating the main body case 10 near the position of arrangement of the radiation thermometer 50 and opening to the external environment, and also has a reflection mirror 19 arranged in the penetration hole 18. The reflection mirror 19 is supported on the inner side of the penetration hole 18 by a support member 191 attached to the sidewall of the penetration hole 18.

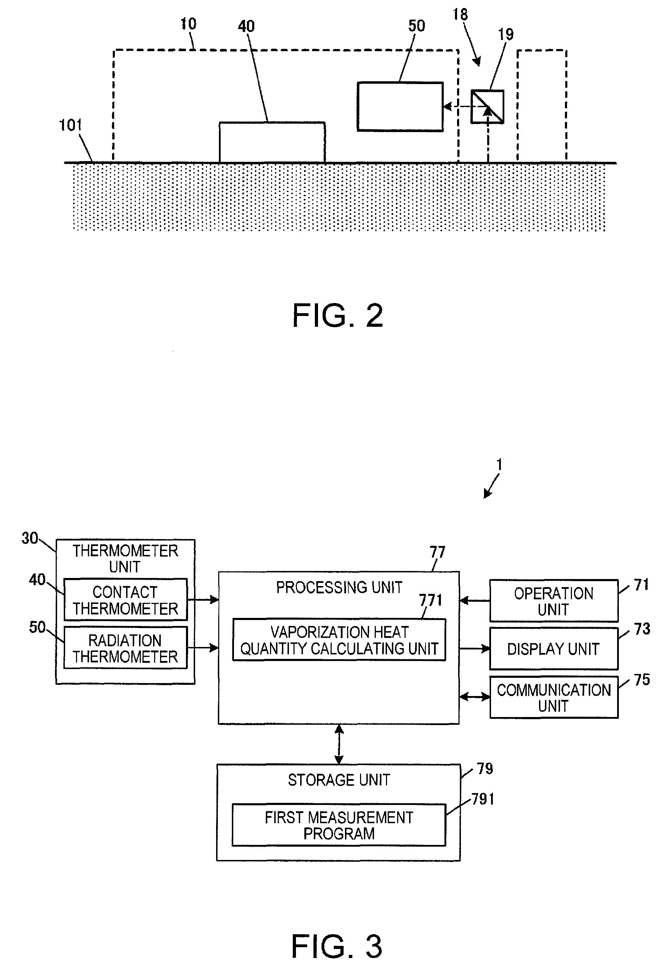

FIG. 2 is a schematic view showing the positional relation between the contact thermometer 40, the radiation thermometer 50 and the reflection mirror 19 on a skin surface 101 of the user 100 when the electronic apparatus 1 is mounted. In FIG. 2, the outer shape of the main body case 10 on its cross section taken along A-A shown in FIG. 1 is indicated by dashed lines. As shown in FIG. 2, the contact thermometer 40 is installed in contact with the skin surface 101 as the electronic apparatus 1 is mounted. The radiation thermometer 50 is arranged in such a way that its height from the skin surface 101 coincides with the height of the reflection mirror 19 when the electronic apparatus 1 is mounted.

The reflection mirror 19 guides infrared rays radiated from the skin surface 101 to the radiation thermometer 50, as indicated by chain-dotted lines with arrows.

Back to FIG. 1, the heat flow meter 60 is configured to measure a heat flow generated on the skin surface 101 of the user 100. The heat flow meter 60 detects the temperature difference between the temperature on the back side of the main body case 10 (the side of the skin surface 101) and the temperature on the face side of the main body case 10 (the external environment side) and measures a heat flow on the basis of the detected heat flow. In the first embodiment, the heat flow meter 60 is not an essential component. However, with the electronic apparatus 1 having the configuration with the heat flow meter 60, the measurement of a heat flow can be carried out along with the measurement of the contact temperature and the radiation temperature at the measuring site (for example, wrist), which is the site where the electronic apparatus 1 is mounted.

Here, the principle of measuring the quantity of heat of perspiration vaporization using the contact temperature (first temperature) measured by the contact thermometer 40 and the radiation temperature (second temperature) measured by the radiation thermometer 50 will be described. When water evaporates by perspiration from the skin surface 101 of the user 100 as a measuring target, heat of vaporization is taken away and therefore the temperature of the skin surface 101 drops. However, as far as the part of the skin surface 101 in contact with the contact thermometer 40 is concerned, since this part is not in contact with the outside air, water does not evaporate and heat of vaporization is not taken away, and therefore the temperature of the skin surface 101 does not drop. That is, if the contact temperature measured by the contact thermometer 40 is Ts and the radiation temperature measured by the radiation thermometer 50 is Tr, the relation between the contact temperature Ts and the radiation temperature Tr expressed by the following equation (1) holds.

$$Ts > Tr \qquad \dots (1)$$

The temperature difference between the contact temperature Ts and the radiation temperature Tr is the temperature difference generated as water evaporates and heat of vaporization is taken away from the skin surface 101, and therefore this temperature different is proportionate to the quantity of heat of perspiration vaporization. The relation between the temperature difference and the quantity of heat of perspiration vaporization can be expressed by the following equation (2), where Q [W] is the quantity of heat of perspiration vaporization per unit time and K is a predetermined constant of proportionality.

$$Q = K(Ts-Tr) \qquad \dots (2)$$

Therefore, the quantity of heat of perspiration vaporization Q can be calculated, that is, measured on the basis of the contact temperature Ts and the radiation temperature Tr, for example, according to the equation (2).

However, the method for measuring the quantity of heat of perspiration vaporization is not limited to this. For example, a true value of the quantity of heat of perspiration vaporization is actually measured by a heat analyzer or the like, and at the same time, the contact temperature and the radiation temperature is measured so as to acquire the relation between the quantity of heat of vaporization, and the contact temperature and the radiation temperature, in advance. When the electronic apparatus 1 is in use, the quantity of heat of perspiration vaporization Q may be calculated by regression analysis based on the contact temperature Ts measured by the contact thermometer 40 and the radiation temperature Tr measured by the radiation thermometer 50. In this case, the quantity of heat of perspiration vaporization Q is calculated on the basis of the linear sum of the contact temperature Ts and the radiation temperature Tr according to the following equation (3), where S and R are predetermined constants of proportionality and D1 is a predetermined constant.

$$Q = S×Ts + R×Tr + D1 \qquad \dots (3)$$

Alternatively, the quantity of heat of perspiration vaporization Q may be calculated by multiple regression analysis based on the contact temperature Ts measured by the contact thermometer 40 and the radiation temperature Tr measured by the radiation thermometer 50. In this case, the quantity of heat of perspiration vaporization Q is calculated on the basis of the sum of squares of the contact temperature Ts and the radiation temperature Tr according to the following equation (4), where S1, S2, R1 and R2 are predetermined constants of proportionality and D2 is a predetermined constant.

$$Q = S1 \times Ts + S2 \times Ts2 + R1 \times Tr + R2 \times Tr2 + D2 \quad \dots (4)$$

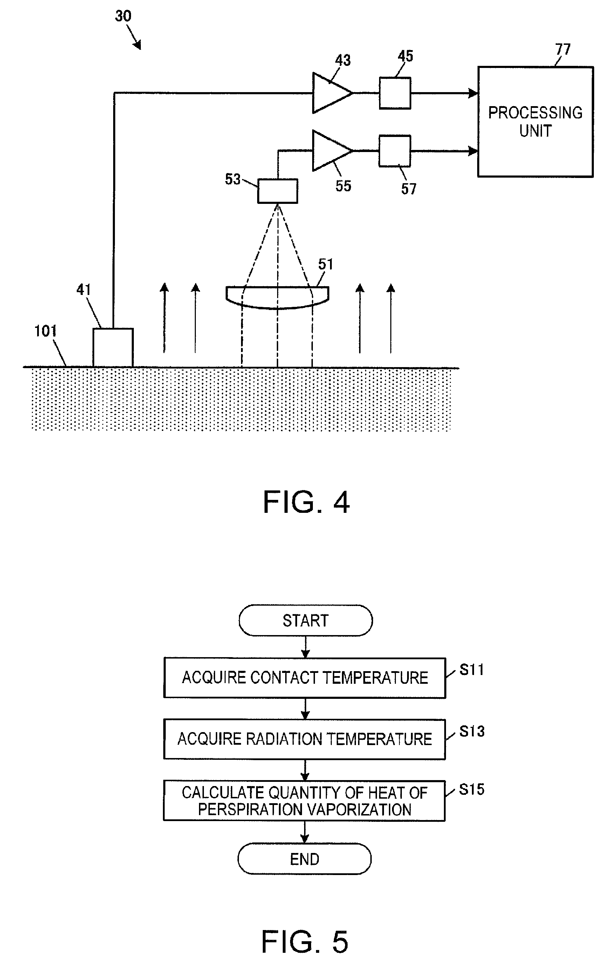

FIG. 3 is a block diagram showing an example of the main functional configuration of the electronic apparatus 1 according to the first embodiment. FIG. 4 is a block diagram schematically showing an example of the internal configuration of the thermometer unit 30. As shown in FIG. 3, the electronic apparatus 1 has the thermometer unit 30, an operation unit 71, a display unit 73, a communication unit 75, a processing unit 77, and a storage unit 79.

The thermometer unit 30 has a contact-type temperature measuring element 41, an amplifier 43, and an AD converter 45, as the configuration of the contact thermometer 40, as shown in FIG. 4. The contact-type temperature measuring element 41 is formed using a thermistor, thermocouple or the like, for example. An output signal from the contact-type temperature measuring element 41 is amplified by the amplifier 43, then AD-converted by the AD converter 45, and outputted as the contact temperature Ts to the processing unit 77 at all times.

The thermometer unit 30 also has an infrared condensing lens 51, a radiation temperature measuring element 53, amplifier 55, and an AD converter 57, as the configuration of the radiation thermometer 50. The infrared condensing lens 51 condenses infrared rays from the skin surface 101 and makes the condensed infrared rays incident on the radiation temperature measuring element 53. Although not illustrated in FIG. 4, the reflection mirror 19 shown in FIG. 1 and the like is suitably arranged according to need, between the infrared condensing lens 51 and the skin surface 101. In this case, the infrared condensing lens 51 is arranged on the optical path of the infrared rays bent by the reflection mirror 19. The radiation temperature measuring element 53 is formed using a thermopile, for example, and detects the intensity of the infrared rays incident thereon via the infrared condensing lens 51. An output signal from the radiation temperature measuring element 53 is amplified by the amplifier 55, then AD-converted by the AD converter 57, and outputted as the radiation temperature Tr to the processing unit 77 at all times.

The operation unit 71 is materialized by various switches such as a button switch, lever switch and dial switch, and an input device such as a touch panel, and outputs an operation signal corresponding to an operation input to the processing unit 77. For example, the operation switch 11 and the touch panel 12 shown in FIG. 1 are equivalent to these.

The display unit 73 is materialized by a display device such as an LCD (liquid crystal display) or EL (electroluminescence display), and displays various screens based on display signals inputted from the processing unit 77. In FIG. 1, the touch panel 12 is equivalent to this. On the display unit 73, the results of measuring the quantity of heat of perspiration vaporization and the like are displayed. For example, the results of measuring the quantity of heat of perspiration vaporization are displayed in the form of a current vaporization heat quantity display screen, or a heat vaporization heat quantity display screen showing changes in the quantity of heat of perspiration vaporization as a graph based on past logging data, or the like, in response to a display mode switch operation on the operation unit 71.

The communication unit 75 is a communication device for transmitting and receiving information used inside the device to and from an external information processing device, under the control of the processing unit 77. The communication device 13 in FIG. 1 is equivalent to this. As the communication method of the communication unit 75, various methods can be used, such as wired connection via a cable conforming to a predetermined communication standard, connection via an intermediate device which also functions as a charger, called cradle, or wireless connection utilizing wireless communication.

The processing unit 77 is a control device and computing device which centrally controls each part of the electronic apparatus 1, and is materialized by a microprocessor such as a CPU (central processing unit) or GPU (graphic processing unit), and an ASIC (application specific integrated circuit), FPGA (field-programmable gate array), IC (integrated circuit) memory or the like. In FIG. 1, the CPU 171 on the control board 17 is equivalent to this. The processing unit 77 includes a vaporization heat quantity calculating unit 771 which calculates the quantity of heat of perspiration vaporization, using the contact temperature Ts inputted form the contact thermometer 40 and the radiation temperature Tr inputted from the radiation thermometer 50.

The storage unit 79 is materialized by a storage medium including various IC memories such as a ROM (read only memory), flash ROM, and RAM (random access memory), and a hard disk or the like, and suitably includes a device for reading and writing data according to need. In FIG. 1, the storage medium 173 on the control board 17, the memory card 15, and the reader/writer 14 are equivalent to this. In the storage unit 79, a program for implement various functions provided in the electronic apparatus 1 and data or the like used during execution of the program are stored in advance, or temporarily stored every time processing is performed. In the storage unit 79, a first measurement program 791 for causing the processing unit 77 to function as the vaporization heat quantity calculating unit 771 and executing vaporization heat quantity measurement processing is stored as well.

FIG. 5 is flowchart showing the flow of the vaporization heat quantity measurement processing according to the first embodiment. The processing described here can be implemented by the processing unit 77 reading and executing the first measurement program 791 from the storage unit 79.

In this vaporization heat quantity measurement processing, the vaporization heat quantity calculating unit 771 first acquires the contact temperature Ts from the contact thermometer 40 (Step S11) and acquires the radiation temperature Tr from the radiation thermometer 50 (step S13).

Then, the vaporization heat quantity calculating unit 771 executes computing processing of substituting the contact temperature Ts acquired in Step S11 and the radiation temperature Tr acquired in Step S13 in the equation (2) and thus calculating the quantity of heat of perspiration vaporization Q, as vaporization heat quantity computing processing (Step S15). Computing processing using the equation (3) or the equation (4) or the like to calculate the quantity of heat of perspiration vaporization Q may be executed as vaporization heat quantity computing processing. The quantity of heat of perspiration vaporization Q calculated here is displayed on the display unit 73 according to need.

As described above, according to the first embodiment, the quantity of heat of perspiration vaporization can be calculated using the contact temperature and the radiation temperature measured as the temperature of the skin surface of the user 100, and the quantity of heat of perspiration vaporization from the user 100 can be measured. Thus, even in environments where perspiration occurs, for example, at the time of exercise or in a hot environment or the like, the quantity of heat of perspiration vaporization can be measured accurately.

In the first embodiment, the case where the measuring target is a human body whose quantity of heat of perspiration vaporization is measured is described. However, the technique can similarly applied to the case where the measuring target is other animals than human and where the quantity of heat of vaporization due to perspiration of the animals is measured, or the case where the measuring target is an object which generates heat of vaporization (for example, a vegetable or fruit) and where the quantity of heat of vaporization from the object is measured.

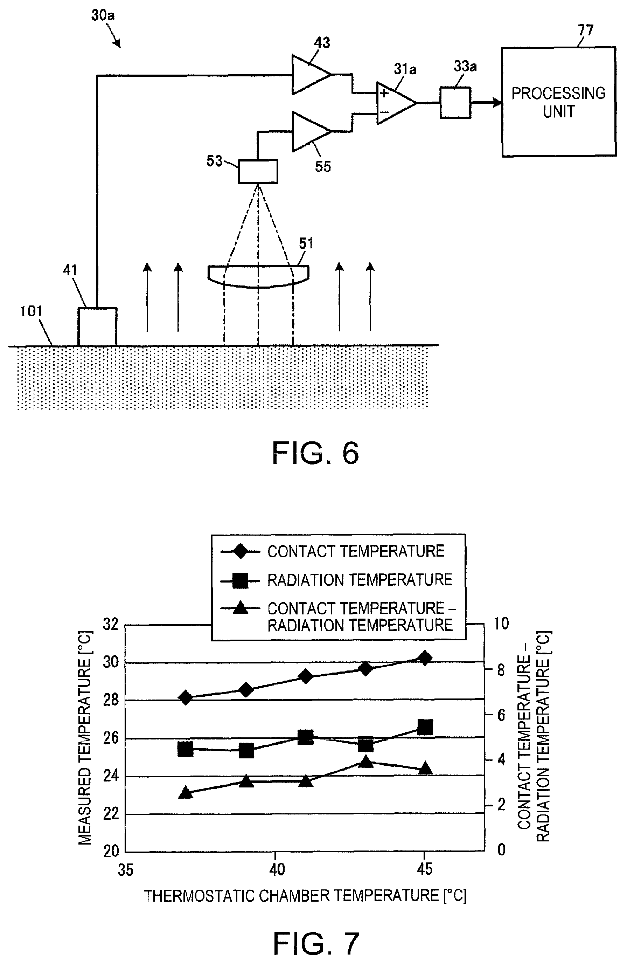

The internal configuration of the thermometer unit 30 is not limited to the configuration shown in FIG. 4. FIG. 6 is a block diagram schematically showing an example of the internal configuration of a thermometer unit 30a according to a modification. In FIG. 6, the same components as in the configuration of FIG. 4 are denoted by the same reference numbers. The thermometer unit 30a shown in FIG. 6 has a differential amplifier 31a which performs differential amplification of the contact temperature Ts and the radiation temperature Tr, and an AD converter 33a, subsequent to the amplifiers 43, 55. The temperature difference between the contact temperature Ts and the radiation temperature Tr is outputted to the processing unit 77 at all times. In the case where the configuration of the thermometer unit 30a according to this modification is employed, the vaporization heat quantity calculating unit 771 of the processing unit 77 performs, for example, computing processing of multiplying the temperature difference between the contact temperature Ts and the radiation temperature Tr inputted form the thermometer unit 30a by the constant of proportionality K in the equation (2), as vaporization heat quantity computing processing, and thus calculates the quantity of heat of perspiration vaporization Q.

Meanwhile, the measurement of the quantity of heat of perspiration vaporization according to the first embodiment is configured on the assumption that a temperature difference occurs between the contact temperature and the radiation temperature of the skin surface due to perspiration (more specifically, the radiation temperature is lower than the contact temperature). In order to confirm this assumption, an experiment to prove that a temperature difference occurs between the contact temperature and the radiation temperature due to the heat of vaporization being taken away from the surface of a measuring target is carried out. In the experiment, a thermostatic chamber is prepared as a heat source and a nonwoven fabric impregnated with water is used as a measuring target. Specifically, while the nonwoven fabric is heated stepwise (for example, by 2 [°C] each) within the range of 37.0 [°C] to 45.0 [°C] by controlling the temperature of the thermostatic chamber, the contact thermometer is brought in contact with a part of the nonwoven fabric and the contact temperature is thus measured, and at the same time, the surface temperature of the nonwoven fabric is remotely measured as the radiation temperature, using thermography.

FIG. 7 shows the results of the experiment. In FIG. 7, change in the contact temperature and change in the radiation temperature measured with each temperature of the thermostatic chamber are shown, and the temperature difference between the contact temperature and the radiation temperature measured with each temperature of the thermostatic chamber (contact temperature - radiation temperature) is shown as well. As shown by the results of the experiment in FIG. 7, this experiment successfully confirms that a temperature difference occurs between the contact temperature and the radiation temperature due to the evaporation of water from the surface of the measuring target and that the radiation temperature is lower than the contact temperature. Also, though not illustrated, it is successfully confirmed from the distribution of the radiation temperature on the surface of the nonwoven fabric obtained by thermography that the temperature at the site of contact of the contact temperature is higher than in the other areas.

Second Embodiment

Next, a second embodiment will be described. In the second embodiment, the metabolic rate due to perspiration of the user 100 is measured, using the quantity of heat of perspiration vaporization found in the first embodiment. In the description below, the same components as in the configuration of the first embodiment are denoted by the same reference numbers.

If the quantity of heat of perspiration vaporization is expressed by Qsw, the metabolic rate due to perspiration Csw can be found by multiplying the quantity of heat of perspiration vaporization Qsw by a predetermined constant of proportionality Ka, as expressed by the following equation (5).

$$\mathrm{Csw \; = \; Ka \times Qsw} \qquad \ldots \; (5)$$

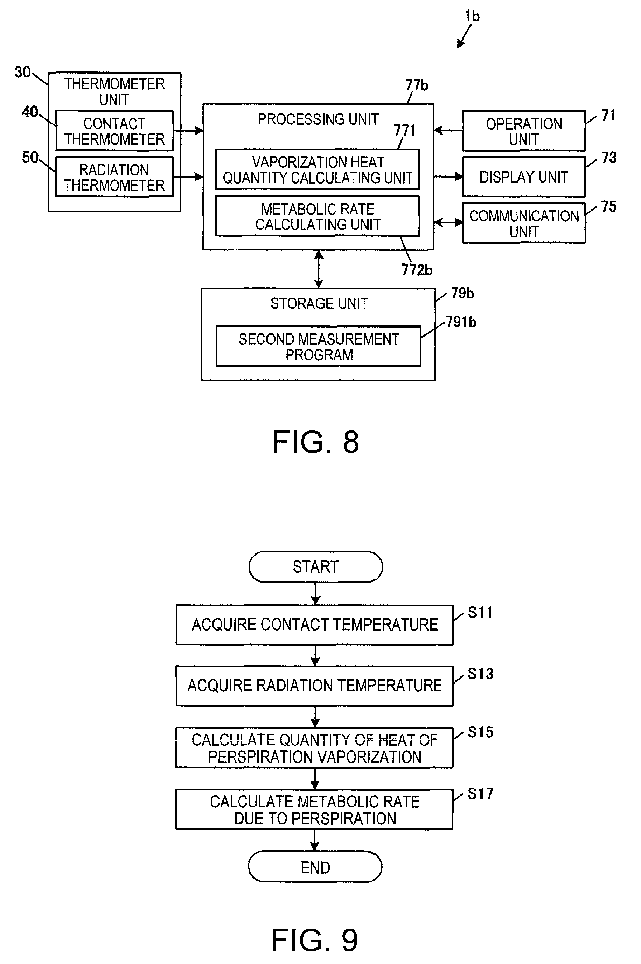

The constant of proportionality Ka varies depending on the site for measuring the quantity of heat of perspiration vaporization and its surface area, and also depending on the measuring conditions such as the weight, height, age and gender of the user 100. The degree of perspiration (quantity of perspiration) varies from one part to another of the entire body and also changes according to the weight or the like of the user 100. Thus, parent sets in which the measuring conditions such as weight are different are selected, for example. Then, the quantity of heat of perspiration vaporization is measured by the method according to the first embodiment, for example, and at the same time, the true value of the metabolic rate is actually measured by a respiratory gas analyzer. Based on this statistical value, the constant of proportionality Ka is found for each parent set. Thus, the constant of proportionality Ka corresponding to the measuring conditions is set in advance. When an electronic apparatus 1b (see FIG. 8) is in use, an input operation to input each measuring condition item such as weight by the user 100 is accepted at the beginning, and the constant of proportionality Ka to be used for the calculation of the metabolic rate due to perspiration Csw is decided on the basis of the inputted value of each measuring condition item. Measurement is carried out subsequently.

FIG. 8 is a block diagram showing an example of the main functional configuration of the electronic apparatus 1b according to the second embodiment. As shown in FIG. 8, the electronic apparatus 1b has a thermometer unit 30, an operation unit 71, a display unit 73, a communication unit 75, a processing unit 77b, and a storage unit 79b.

In the second embodiment, the processing unit 77b includes a vaporization heat quantity calculating unit 771, and a metabolic rate calculating unit 772b which calculates the metabolic rate due to perspiration, using the quantity of heat of perspiration vaporization. In the storage unit 79b, a second measurement program 791b for causing the processing unit 77b to function as the vaporization heat quantity calculating unit 771 and the metabolic rate calculating unit 772b and execute metabolic rate measurement processing is stored.

FIG. 9 is a flowchart showing the flow of the metabolic rate measurement processing according to the second embodiment. The processing described here can be implemented by the processing unit 77b reading and executing the second measurement program 791b from the storage unit 79b. In FIG. 9, the same steps as in the first embodiment are denoted by the same reference numbers.

In this metabolic rate measurement processing, following Step S15, the metabolic rate calculating unit 772b substitutes the quantity of heat of perspiration vaporization Q calculated in Step S15 as Qsw in the equation (5) and thus calculates the metabolic rate due to perspiration Csw (Step S17). The metabolic rate due to perspiration Csw calculated here is displayed on the display unit 73 together with the quantity of heat of perspiration vaporization Q (Qsw) according to need.

As described above, according to the second embodiment, the metabolic rate due to perspiration of the user 100 can be measured, using the quantity of heat of perspiration vaporization found by measuring the contact temperature and the radiation temperature of the skin surface of the user 100.

Third Embodiment

Next, a third embodiment will be described. In the description below, the same components as in the configuration according to the second embodiment are denoted by the same reference numbers.

In the second embodiment, the metabolic rate due to perspiration is found on the basis of the quantity of heat of perspiration vaporization measured at one measuring site. However, if the quantity of heat of perspiration vaporization is measured at different measuring sites on the user 100, the metabolic rate due to perspiration can be measured with higher accuracy. The metabolic rate due to perspiration Csw in this case is expressed by the following equation (6).

$$\mathrm{Csw \; = \; Kb1 \times Qsw1 \; + \; Kb2 \times Qsw2 \; + \; \ldots \; + \; Kbn \times Qswn} \qquad \ldots \; (6)$$

Here, in the equation (6), Qsw1, Qsw2, ..., Qswn represent the quantities of heat of perspiration vaporization Qsw at the measuring sites identified by the added numbers 1, 2, ..., n. Also, Kb1, Kb2, ..., Kbn represent predetermined constants of proportionality set in advance for the respective measuring sites identified by the added numbers 1, 2, ..., n. The reason for setting the constants of proportionality Kb1, Kb2, ..., Kbn for the respective measuring sites is that the degree of perspiration varies from one site to another on the entire body and also changes according to the surface area or the like of the measuring site. For example, the quantity of heat of perspiration vaporization is measured at each measuring site by the method according to the first embodiment, and at the same time, the true value of the metabolic rate is actually measured by a respiratory gas analyzer. Then, multivariate analysis such as multiple regression analysis is performed, thus finding and setting the constants of proportionality Kb1, Kb2, ..., Kbn.

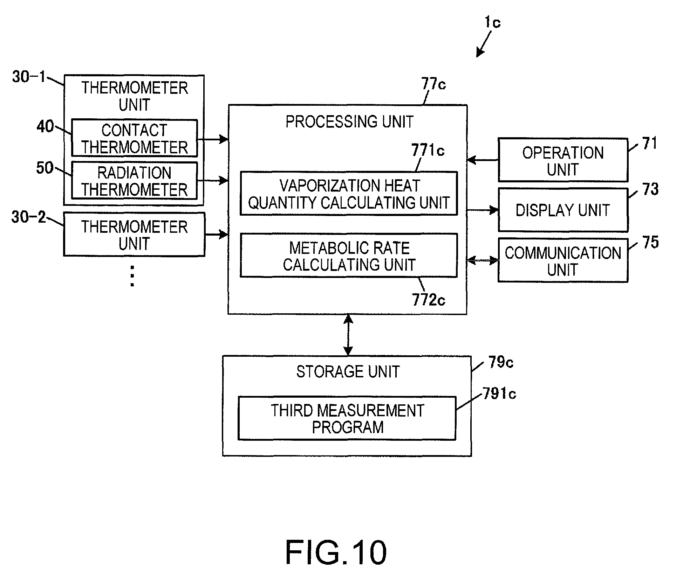

FIG. 10 is a block diagram showing an example of the main functional configuration of an electronic apparatus 1c according to the third embodiment. As shown in FIG. 10, the electronic apparatus 1c has a plurality of thermometer units 30 (30-1, 2, ..., n), an operation unit 71, a display unit 73, a communication unit 75, a processing unit 77c, and a storage unit 79c.

The plurality of thermometer units 30 (30-1, 2, ..., n) are mounted at different measuring sites on the user 100 and measure the contact temperature Ts and the radiation temperature Tr on the skin surface. The configuration of each individual thermometer unit 30 can be formed similarly to the configuration described in the first embodiment. As a specific method for materialization, for example, apart from the electronic apparatus 1c having one thermometer unit 30 similarly to the apparatus shown in FIG. 1, one or a plurality of measuring apparatuses, each having a main body case with a thermometer unit 30, a reflection mirror 19, a communication device 13, a reader/writer 14 or the like similarly arranged therein and having a band provided thereon, are prepared and these are mounted and fixed at target measuring sites in such a direction that the contact temperature 40 contacts the skin surface. In this case, each of the plurality of thermometer units 30, with a vaporization heat quantity calculating unit 771c, forms a vaporization heat quantity measuring device. The exchange of the results of measurement between the individual apparatuses may be carried out via the communication device 13 and the reader/writer 14.

The processing unit 77c includes a vaporization heat quantity calculating unit 771c and a metabolic rate calculating unit 772c. In the storage unit 79c, a third measurement program 791c is stored.

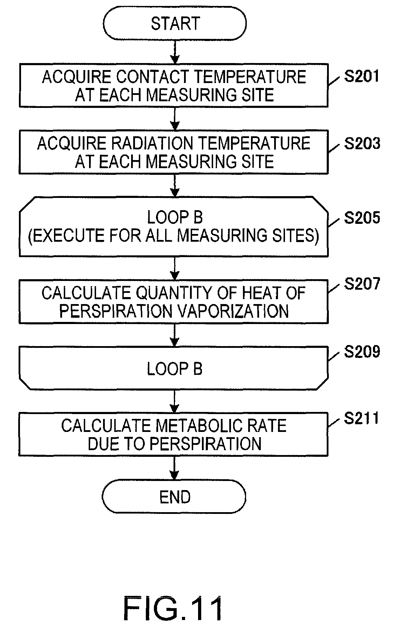

FIG. 11 is a flowchart showing the flow of the metabolic rate measurement processing according to the third embodiment. The processing described here can be implemented by the processing unit 77c reading and executing the third measurement program 791c from the storage unit 79c.

In the metabolic rate measurement processing according to the third embodiment, first, the vaporization heat quantity calculating unit 771c acquires the contact temperature Ts measured by each contact thermometer 40 at each measuring site (Step S201) and also acquires the radiation temperature Tr measured by each radiation thermometer 50 at each measuring site (Step S203).

Subsequently, the vaporization heat quantity calculating unit 771c executes processing of loop B, sequentially taking the measuring sites where the respective thermometer units 30 are installed, as processing target sites (Step S205 to Step S209). That is, in the loop B, the vaporization heat quantity calculating unit 771c calculates the quantity of heat of perspiration vaporization Q at the processing target site, for example, according to the equation (2) using the contact temperature Ts and the radiation temperature Tr acquired from the contact thermometer 40 and the radiation thermometer 50 at the processing target site in Step S201 and Step S203 (Step S207).

Then, after the quantities of heat of perspiration vaporization Q at all the measuring sites are calculated, the metabolic rate calculating unit 772c substitutes the quantity of heat of perspiration vaporization Q at each measuring site calculated in Step S207, as Qsw1, Qsw2, ..., Qswn in the equation (6) and thus calculates the metabolic rate due to perspiration Csw (Step S211). The metabolic rate due to perspiration Csw calculated here is displayed on the display unit 73 together with the quantity of heat of perspiration vaporization Q (Qsw1, Qsw2, ..., Qswn) at each measuring site according to need.

As described above, according to the third embodiment, the quantity of heat of perspiration vaporization is measured at different measuring sites on the user 100, and the metabolic rate to due perspiration can be calculated from the quantity of heat of perspiration vaporization at each measuring site. Therefore, the accuracy of measurement of the metabolic rate due to perspiration of the user 100 is improved.

Fourth Embodiment

Next, a fourth embodiment will be described. In the fourth embodiment, the metabolic rate due to heat flow is calculated apart from the metabolic rate due to perspiration, and the total metabolic rate of the user 100 is measured. In the description below, the same components as in the configuration according to the third embodiment are denoted by the same reference numbers.

Heat dissipation from a human body includes heat dissipation generated by the perspiration function (evaporative heat loss) and heat dissipation due to heat transfer (convection), radiation of electromagnetic waves, and the like (non-evaporative heat loss). Therefore, the metabolic rate can be measured with higher accuracy by taking both into account.

The heat dissipation from a human body due to heat transfer and the like can be measured as a heat flow generated on the skin surface of the user 100. For example, in the case of measuring the heat flow at a plurality of measuring sites, the metabolic rate due to heat flow Cc can be found by the following equation (7).

$$Cc = L1 \times Qc1 + L2 \times Qc2 + \ldots + Ln \times Qcn \qquad \ldots (7)$$

Here, in the equation (7), Qc1, Qc2, ..., Qcn represent the heat flows Qc identified by the added numbers 1, 2, ..., n. L1, L2, ..., Ln represent constants of proportionality set in advance for the respective measuring sites identified by the added numbers 1, 2, ..., n. The reason for setting the constants of proportionality L1, L2, ..., Ln for the respective measuring sites is that the heat dissipation from the human body due to heat transfer is not uniform at each site on the entire body and also varies depending on the area of the measuring site and the like. For example, the true value of the metabolic rate is actually measured by a respiratory gas analyzer, and at the same time, the heat flow at each measuring site is measured. Then, multivariate analysis such as multiple regression analysis is performed. The constants of proportionality L1, L2, ..., Ln are thus found and set.

The total metabolic rate C can be found by the following equation (8) based on the metabolic rate due to perspiration Csw and the metabolic rate due to heat flow Cc. In the equation (8), Asw and Ac are predetermined constants of proportionality.

$$C = AswCsw + AcCc \qquad \ldots (8)$$

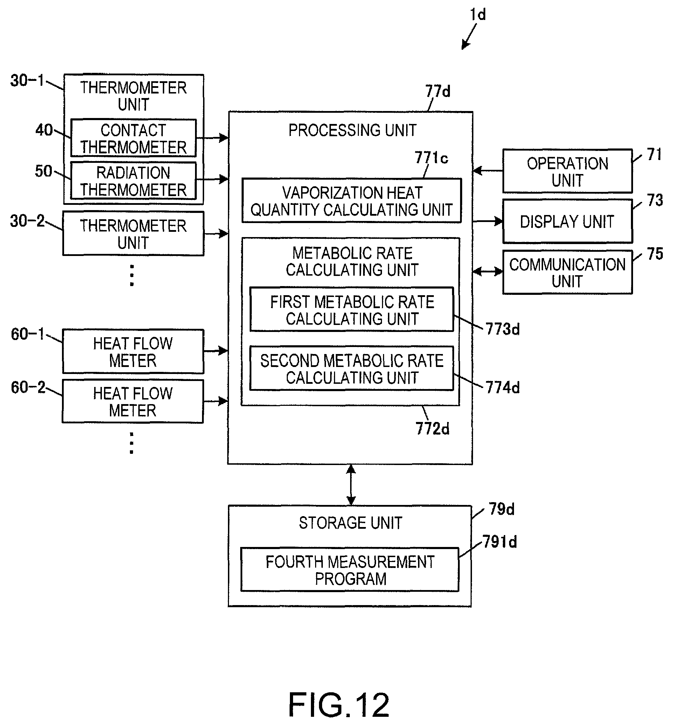

FIG. 12 is a block diagram showing an example of the main functional configuration of an electronic apparatus 1d according to the fourth embodiment. As shown in FIG. 12, the electronic apparatus 1d has a plurality of thermometer units 30 (30-1, 2, ..., n), a plurality of heat flow meters 60 (60-1, 2, ..., n), an operation unit 71, a display unit 73, a communication unit 75, a processing unit 77d, and a storage unit 79d.

The plurality of thermometer units 30 (30-1, 2, ..., n) measure the contact temperature Ts and the radiation temperature Tr of the skin surface at different measuring sites on the user 100. Meanwhile, the plurality of heat flow meters 60 (60-1, 2, ..., n) measure the heat flows Qc1, Qc2, ..., Qcn generated on the skin surface at different measuring sites on the user 100. As a specific method for implementation, for example, the electronic apparatus 1d having one thermometer unit 30 as in the configuration described in FIG. 1 and having one heat flow meter 60, and one or a plurality of measuring apparatuses configured as described in the third embodiment and also having a heat flow meters 60, are prepared, and these apparatuses are mounted and fixed at the target measuring sites in such a direction that the contact thermometer 40 contacts the skin surface. However, this is not limiting and the thermometer unit 30 and the heat flow meter 60 may be separate units, for example.

The processing unit 77d includes a vaporization heat quantity calculating unit 771c and a metabolic rate calculating unit 772d. The metabolic rate calculating unit 772d includes a first metabolic rate calculating unit 773d which calculates the metabolic rate due to perspiration, and a second metabolic rate calculating unit 774d which calculates the metabolic rate to heat flow, using the heat flows Qc1, Qc2, ..., Qcn inputted from the heat flow meters 60 at the respective measuring sites. The metabolic rate calculating unit 772d thus calculates the total metabolic rate based on the metabolic rate due to perspiration and the metabolic rate due to heat flow. In the storage unit 79d, a fourth measurement program 791d is stored.

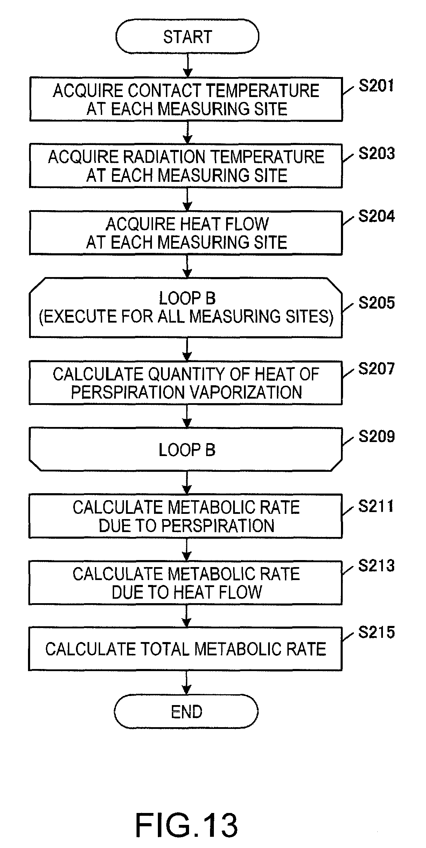

FIG. 13 is a flowchart showing the flow of the metabolic rate calculation processing according to the fourth embodiment. The processing described here can be implemented by the processing unit 77d reading and executing the fourth measurement program 791d from the storage unit 79d. In FIG. 13, the same steps as in the third embodiment are denoted by the same reference numbers.

In the metabolic rate calculation processing according to the fourth embodiment, after Step S203, the metabolic rate calculating unit 772d acquires the heat flows Qc1, Qc2, ..., Qcn from the plurality of heat flow meters 60 (step S204).

After the processing of loop B, the first metabolic rate calculating unit 773d calculates the metabolic rate due to perspiration Csw according to the equation (6) (Step S211). Subsequently, the second metabolic rate calculating unit 774d substitutes the heat flows Qc1, Qc2, ..., Qcn at the respective measuring sites acquired in Step S204, in the equation (7), and thus calculates the metabolic rate due to heat flow Cc (step S213). Then, the metabolic rate calculating unit 772d substitutes the metabolic rate due to perspiration Csw calculated in Step S211 and the metabolic rate due to heat flow Cc calculated in Step S213, in the equation (8), and thus calculates the total metabolic rate C (Step S215). The total metabolic rate C calculated here is displayed on the display unit 73 together with the quantities of heat of perspiration vaporization Q (Qsw1, Qsw2, ..., Qswn) at the respective measuring sites calculated in Step S207, the metabolic rate due to perspiration Csw and the metabolic rate due to heat flow Cc, according to need.

As described above, according to the fourth embodiment, the heat flow generated in the skin surface is measured at different measuring sites on the user 100, and the metabolic rate due to heat flow of the user 100 can be calculated. Then, the total metabolic rate can be calculated on the basis of the metabolic rate due to perspiration and the metabolic rate due to heat flow. Thus, the total metabolic rate can be found, taking into account not only the heat dissipated as heat of vaporization from the skin surface due to perspiration but also the heat dissipated from the skin surface due to heat transfer or the like. Therefore, the metabolic rate of the user 100 can be measured with higher accuracy.

While the case of measuring the quantity of heat of perspiration vaporization and the heat flow at a plurality of measuring sites is described in the fourth embodiment, a single measuring site may be used. That is, the metabolic rate due to perspiration may be found on the basis of the quantity of heat of perspiration vaporization measured at a single measuring site, and the metabolic rate due to heat flow may be found on the basis of the heat flow measured at a single measuring site, so as to calculate the total metabolic rate.

In the embodiments, the measurement of the quantity of heat of perspiration vaporization, and the measurement of the metabolic rate using the quantity of heat of perspiration vaporization and the heat flow are described. However, it is also possible to measure biological information other than metabolic rate, for example, to find the calories burned, using the quantity of heat of perspiration vaporization and the heat flow, or to measure the functions of autonomic nerves of the human body.

**Claims**

1. A vaporization heat quantity measuring device comprising:

   a contact thermometer adapted to contact a surface of a measuring target and to measure a first temperature of the surface;
   a radiation thermometer adapted to measure a second temperature of the surface of the measuring object; and
   a computing unit adapted to calculate a quantity of heat of vaporization, using the first temperature and the second temperature.

2. The vaporization heat quantity measuring device according to claim 1, wherein the computing unit is adapted to execute predetermined vaporization heat quantity computing processing in which the first temperature and the second temperature are variables, and thus to calculate the quantity of heat of vaporization.

3. The vaporization heat quantity measuring device according to claim 2, wherein the computing unit is adapted to execute computing processing in which the quantity of heat of vaporization is calculated on the basis of a linear sum of the first temperature and the second temperature, as the vaporization heat quantity computing processing.

4. The vaporization heat quantity measuring device according to claim 2, wherein the computing unit is adapted to execute computing processing in which the quantity of heat of vaporization is calculated on the basis of a sum of squares of the first temperature and the second temperature, as the vaporization heat quantity computing processing.

5. A biological information measuring device comprising a plurality of the vaporization heat quantity measuring devices according to any one of the preceding claims which is adapted to measure the quantity of heat of vaporization at different sites on a living body as the measuring target,
   wherein the biological information measuring device is adapted to measure biological information including at least a metabolic rate of the living body, using the quantity of heat of vaporization measured at each of the sites.

6. A biological information measuring device comprising:

   the vaporization heat quantity measuring device according to any one of the preceding claims which is adapted to measure the quantity of heat of vaporization from a living body as the measuring target; and
   a heat flow meter which is adapted to measure a heat flow generated on a surface of the living body;
   wherein the biological information measuring device is adapted to measure biological information including at least a metabolic rate of the living body, using the quantity of heat of vaporization measured by the vaporization heat quantity measuring device and the heat flow measured by the heat flow meter.

7. An electronic apparatus adapted to be mounted on a skin surface at a predetermined site of a user by having a band thereof wound on the site, the electronic apparatus comprising:

   a contact thermometer adapted to contact the skin surface and to measure a first temperature of the skin surface;
   a radiation thermometer adapted to measure a second temperature of the skin surface; and
   a computing unit which adapted to calculate a quantity of heat of vaporization, using the first temperature and the second temperature.

[ FACE SIDE ]

[ BACK SIDE ]

FIG. 1

10  40  50  18  19

101

# FIG. 2

1

30 — THERMOMETER UNIT

40 — CONTACT THERMOMETER

50 — RADIATION THERMOMETER

77 — PROCESSING UNIT

771 — VAPORIZATION HEAT QUANTITY CALCULATING UNIT

71 — OPERATION UNIT

73 — DISPLAY UNIT

75 — COMMUNICATION UNIT

79 — STORAGE UNIT

791 — FIRST MEASUREMENT PROGRAM

# FIG. 3

# FIG. 4

START

ACQUIRE CONTACT TEMPERATURE — S11

ACQUIRE RADIATION TEMPERATURE — S13

CALCULATE QUANTITY OF HEAT OF PERSPIRATION VAPORIZATION — S15

END

# FIG. 5

FIG. 6

FIG. 7

1b

```
                                                              77b
┌─────────────────┐    ┌─────────────────────────┐    ┌──────────────┐
│ 30  THERMOMETER │    │      PROCESSING UNIT    │    │  OPERATION   │ 71
│     UNIT        │    │                    771  │◄───│    UNIT      │
│ 40  ┌─────────┐ │    │ ┌─────────────────────┐ │    └──────────────┘
│     │ CONTACT │ │───►│ │  VAPORIZATION HEAT  │ │    ┌──────────────┐
│     │THERMOMET│ │    │ │QUANTITY CALCULATING │ │───►│ DISPLAY UNIT │ 73
│     │   ER    │ │    │ │        UNIT         │ │    └──────────────┘
│ 50  ├─────────┤ │    │ ├─────────────────────┤ │    ┌──────────────┐
│     │RADIATION│ │───►│ │   METABOLIC RATE    │ │◄──►│COMMUNICATION │ 75
│     │THERMOMET│ │    │ │  CALCULATING UNIT   │ │    │    UNIT      │
│     │   ER    │ │    │ └─────────────────────┘ │    └──────────────┘
│     └─────────┘ │    └─────────────────────────┘
└─────────────────┘                    772b
                               ▲
                               │
                               ▼
                    ┌─────────────────────────┐
                    │      STORAGE UNIT       │ 79b
                    │ ┌─────────────────────┐ │ 791b
                    │ │ SECOND MEASUREMENT  │ │
                    │ │      PROGRAM        │ │
                    │ └─────────────────────┘ │
                    └─────────────────────────┘
```

# FIG. 8

```
        ┌─────────────┐
        │    START    │
        └─────────────┘
               │
               ▼
   ┌─────────────────────────┐
   │ ACQUIRE CONTACT         │ S11
   │ TEMPERATURE             │
   └─────────────────────────┘
               │
               ▼
   ┌─────────────────────────┐
   │ ACQUIRE RADIATION       │ S13
   │ TEMPERATURE             │
   └─────────────────────────┘
               │
               ▼
   ┌─────────────────────────┐
   │ CALCULATE QUANTITY OF   │ S15
   │ HEAT OF PERSPIRATION    │
   │ VAPORIZATION            │
   └─────────────────────────┘
               │
               ▼
   ┌─────────────────────────┐
   │ CALCULATE METABOLIC     │ S17
   │ RATE DUE TO             │
   │ PERSPIRATION            │
   └─────────────────────────┘
               │
               ▼
        ┌─────────────┐
        │     END     │
        └─────────────┘
```

# FIG. 9

FIG.10

START

ACQUIRE CONTACT TEMPERATURE
AT EACH MEASURING SITE — S201

ACQUIRE RADIATION TEMPERATURE
AT EACH MEASURING SITE — S203

LOOP B
(EXECUTE FOR ALL MEASURING SITES) — S205

CALCULATE QUANTITY OF HEAT OF
PERSPIRATION VAPORIZATION — S207

LOOP B — S209

CALCULATE METABOLIC RATE
DUE TO PERSPIRATION — S211

END

# FIG.11

1d

30-1 — THERMOMETER UNIT

40 — CONTACT THERMOMETER

50 — RADIATION THERMOMETER

30-2 — THERMOMETER UNIT

⋮

60-1 — HEAT FLOW METER

60-2 — HEAT FLOW METER

⋮

77d

PROCESSING UNIT

771c

VAPORIZATION HEAT QUANTITY CALCULATING UNIT

METABOLIC RATE CALCULATING UNIT

FIRST METABOLIC RATE CALCULATING UNIT — 773d

SECOND METABOLIC RATE CALCULATING UNIT — 774d

772d

OPERATION UNIT — 71

DISPLAY UNIT — 73

COMMUNICATION UNIT — 75

STORAGE UNIT — 79d

FOURTH MEASUREMENT PROGRAM — 791d

FIG.12

```
                    ┌─────────────────┐
                    │      START      │
                    └────────┬────────┘
                             │
            ┌────────────────▼────────────────┐
            │  ACQUIRE CONTACT TEMPERATURE     │ ╱ S201
            │     AT EACH MEASURING SITE       │
            └────────────────┬────────────────┘
                             │
            ┌────────────────▼────────────────┐
            │  ACQUIRE RADIATION TEMPERATURE   │ ╱ S203
            │     AT EACH MEASURING SITE       │
            └────────────────┬────────────────┘
                             │
            ┌────────────────▼────────────────┐
            │        ACQUIRE HEAT FLOW         │ ╱ S204
            │     AT EACH MEASURING SITE       │
            └────────────────┬────────────────┘
                             │
            ┌────────────────▼────────────────┐
            │            LOOP B                │ ╱ S205
            │ (EXECUTE FOR ALL MEASURING SITES)│
            └────────────────┬────────────────┘
                             │
            ┌────────────────▼────────────────┐
            │  CALCULATE QUANTITY OF HEAT OF   │ ╱ S207
            │    PERSPIRATION VAPORIZATION     │
            └────────────────┬────────────────┘
                             │
            ┌────────────────▼────────────────┐
            │            LOOP B                │ ╱ S209
            └────────────────┬────────────────┘
                             │
            ┌────────────────▼────────────────┐
            │     CALCULATE METABOLIC RATE     │ ╱ S211
            │        DUE TO PERSPIRATION       │
            └────────────────┬────────────────┘
                             │
            ┌────────────────▼────────────────┐
            │     CALCULATE METABOLIC RATE     │ ╱ S213
            │        DUE TO HEAT FLOW          │
            └────────────────┬────────────────┘
                             │
            ┌────────────────▼────────────────┐
            │  CALCULATE TOTAL METABOLIC RATE  │ ╱ S215
            └────────────────┬────────────────┘
                             │
                    ┌────────▼────────┐
                    │       END       │
                    └─────────────────┘
```

# FIG.13

Europäisches
Patentamt

European
Patent Office

Office européen
des brevets

## EUROPEAN SEARCH REPORT

**Application Number**

EP 16 15 4055

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/173730 A1 (POTTGEN PAUL A [US] ET AL) 21 November 2002 (2002-11-21) * paragraphs [0039] - [0042], [0055] - [0059], [0065] - [0066], [0068]; claims; figures * | 1-7 | INV. A61B5/00 A61B5/01 |
| | ----- | | ADD. |
| X | EP 1 774 902 A1 (HITACHI LTD [JP]) 18 April 2007 (2007-04-18) * paragraphs [0014], [0028], [0077] - [0103]; claims; figures * | 1-7 | G01K17/02 G01K17/12 G01K17/20 G01K13/00 G01J5/02 |
| | ----- | | |
| X | US 2014/206955 A1 (STIVORIC JOHN M [US] ET AL) 24 July 2014 (2014-07-24) * paragraphs [0152] - [0161], [0237] - [0238]; claims; figures 22-27,48; table 3 * | 1-7 | |
| | ----- | | |
| X | US 2013/131519 A1 (LEBOEUF STEVEN FRANCIS [US] ET AL) 23 May 2013 (2013-05-23) * paragraphs [0144], [0179] - [0181], [0199] - [0200], [0233]; claims; figures * | 1-7 | TECHNICAL FIELDS SEARCHED (IPC) |
| | ----- | | A61B G01K G01J |
| X | US 5 924 996 A (CHO OK KYUNG [DE] ET AL) 20 July 1999 (1999-07-20) * the whole document * | 1-5,7 | |
| | ----- | | |

The present search report has been drawn up for all claims

| Place of search | Date of completion of the search | Examiner |
|---|---|---|
| The Hague | 30 May 2016 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 15 4055

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2016

| Patent document cited in search report | | Publication date | Patent family member(s) | | Publication date |
|---|---|---|---|---|---|
| US 2002173730 | A1 | 21-11-2002 | EP | 1392157 A1 | 03-03-2004 |
| | | | JP | 4298299 B2 | 15-07-2009 |
| | | | JP | 2004528912 A | 24-09-2004 |
| | | | US | 2002173730 A1 | 21-11-2002 |
| | | | WO | 02091917 A1 | 21-11-2002 |
| EP 1774902 | A1 | 18-04-2007 | CN | 1947659 A | 18-04-2007 |
| | | | EP | 1774902 A1 | 18-04-2007 |
| | | | JP | 2007105329 A | 26-04-2007 |
| | | | US | 2007106139 A1 | 10-05-2007 |
| US 2014206955 | A1 | 24-07-2014 | AU | 2003275491 A1 | 04-05-2004 |
| | | | AU | 2003291637 A1 | 04-05-2004 |
| | | | BR | 0315184 A | 30-08-2005 |
| | | | BR | 0315229 A | 30-08-2005 |
| | | | CA | 2501732 A1 | 22-04-2004 |
| | | | CA | 2501899 A1 | 22-04-2004 |
| | | | CA | 2817028 A1 | 22-04-2004 |
| | | | DK | 1551282 T3 | 22-02-2016 |
| | | | EP | 1551281 A2 | 13-07-2005 |
| | | | EP | 1551282 A2 | 13-07-2005 |
| | | | ES | 2562933 T3 | 09-03-2016 |
| | | | JP | 4813058 B2 | 09-11-2011 |
| | | | JP | 4975249 B2 | 11-07-2012 |
| | | | JP | 2006501961 A | 19-01-2006 |
| | | | JP | 2006501965 A | 19-01-2006 |
| | | | KR | 20050055072 A | 10-06-2005 |
| | | | KR | 20050062773 A | 27-06-2005 |
| | | | MX | PA05003686 A | 17-06-2005 |
| | | | MX | PA05003688 A | 30-09-2005 |
| | | | US | 2004133081 A1 | 08-07-2004 |
| | | | US | 2004152957 A1 | 05-08-2004 |
| | | | US | 2008161654 A1 | 03-07-2008 |
| | | | US | 2008161655 A1 | 03-07-2008 |
| | | | US | 2008167536 A1 | 10-07-2008 |
| | | | US | 2008167537 A1 | 10-07-2008 |
| | | | US | 2008167538 A1 | 10-07-2008 |
| | | | US | 2008167539 A1 | 10-07-2008 |
| | | | US | 2008171919 A1 | 17-07-2008 |
| | | | US | 2008171920 A1 | 17-07-2008 |
| | | | US | 2008171921 A1 | 17-07-2008 |
| | | | US | 2008171922 A1 | 17-07-2008 |
| | | | US | 2014206955 A1 | 24-07-2014 |
| | | | US | 2014213855 A1 | 31-07-2014 |
| | | | US | 2014213856 A1 | 31-07-2014 |
| | | | US | 2014213857 A1 | 31-07-2014 |

EPO FORM P0459

For more details about this annex : see Official Journal of the European Patent Office, No. 12/82

## EP 3 053 516 A1

### ANNEX TO THE EUROPEAN SEARCH REPORT
### ON EUROPEAN PATENT APPLICATION NO.

EP 16 15 4055

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2016

| Patent document cited in search report | | | Publication date | Patent family member(s) | | | Publication date |
|---|---|---|---|---|---|---|---|
| | | | | US | 2014221769 | A1 | 07-08-2014 |
| | | | | US | 2014221770 | A1 | 07-08-2014 |
| | | | | US | 2014221774 | A1 | 07-08-2014 |
| | | | | US | 2014222174 | A1 | 07-08-2014 |
| | | | | US | 2014223406 | A1 | 07-08-2014 |
| | | | | US | 2014223407 | A1 | 07-08-2014 |
| | | | | US | 2015245797 | A1 | 03-09-2015 |
| | | | | WO | 2004032715 | A2 | 22-04-2004 |
| | | | | WO | 2004034221 | A2 | 22-04-2004 |
| US 2013131519 | A1 | | 23-05-2013 | US | 2013131519 | A1 | 23-05-2013 |
| | | | | US | 2014249381 | A1 | 04-09-2014 |
| | | | | US | 2014288394 | A1 | 25-09-2014 |
| | | | | US | 2015080741 | A1 | 19-03-2015 |
| | | | | US | 2015119657 | A1 | 30-04-2015 |
| US 5924996 | A | | 20-07-1999 | CA | 2194348 | A1 | 18-01-1996 |
| | | | | CN | 1159151 | A | 10-09-1997 |
| | | | | DE | 4423663 | A1 | 11-01-1996 |
| | | | | EP | 0771168 | A1 | 07-05-1997 |
| | | | | JP | H10503944 | A | 14-04-1998 |
| | | | | KR | 100271095 | B1 | 01-12-2000 |
| | | | | TW | 298563 | B | 21-02-1997 |
| | | | | US | 5924996 | A | 20-07-1999 |
| | | | | WO | 9601075 | A1 | 18-01-1996 |

**EP 3 053 516 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011120917 A